Europäisches Patentamt

European Patent Office

Office européen des brevets

(11) Veröffentlichungsnummer: **0 258 750**
**A2**

(12) **EUROPÄISCHE PATENTANMELDUNG**

(21) Anmeldenummer: 87112090.3

(22) Anmeldetag: 20.08.87

(51) Int. Cl.4: **A61B 5/10**

(30) Priorität: 02.09.86 DE 3629801

(43) Veröffentlichungstag der Anmeldung:
**09.03.88 Patentblatt 88/10**

(84) Benannte Vertragsstaaten:
**AT BE CH DE ES FR GB GR IT LI LU NL SE**

(71) Anmelder: **S + G IMPLANTS GMBH**
**Grapengiesserstrasse 34**
**D-2400 Lübeck(DE)**

(72) Erfinder: **Thomas, Wolfram, Prof. Dr.**
**Poppenbütteler Landstrasse 12**
**D-2000 Hamburg-Poppenbüttel(DE)**
Erfinder: **Holst, Axel, Dr. med.**
**Rübenkamp 148**
**D-2000 HH-Barmbek(DE)**

(74) Vertreter: **Wilcken, Thomas, Dipl.-Ing. et al**
**Musterbahn 1**
**D-2400 Lübeck(DE)**

(54) **Vorrichtung zur Bestimmung von Beinabmessungen.**

(57) Die Erfindung betrifft eine Vorrichtung zur Bestimmung der Abmessungen eines Beines, wie beispielsweise des Abstandes zwischen Gelenkspalten und der Fußfläche. An einem Ende einer Auflageplatte für das Bein ist ein Anschlag für den Fuß vorgesehen, während zumindest an einer Längsseite der Auflageplatte eine Führung für einen Halter sowie eine Meßskala angeordnet sind. Der parallel zur Meßskala verschiebbare Halter trägt einen Meßkopf, so daß je nach Position des auf ein Gelenk oder einen Gelenkspalt auszurichtenden Meßkopfes an der Skala die betreffende Abmessung abgelesen werden kann.

Fig. 1

## Vorrichtung zur Bestimmung von Beinabmessungen

Die Erfindung bezieht sich auf eine Vorrichtung zur Bestimmung der Abmessungen eines Beines, wie beispielsweise des Abstandes zwischen Gelenkspalten und der Fußfläche.

Für das Implantieren von Hüftgelenk-, Kniegelenk-oder Fußgelenk-Endoprothesen ist es erforderlich, die Länge der Extremitäten und die Lage der natürlichen Gelenke festzulegen, um danach die Abmessungen der zu implantierenden Endoprothese bestimmen zu können. Dies geschah bisher durch röntgenologische Meßmethoden, die jedoch nicht sehr genau und aufwendig sind und außerdem eine Belastung des Patietnen durch Strahlung mit sich bringen.

Die Aufgabe der Erfindung besteht darin, Beinlängen sehr genau und ohne Gefahr für den Patienten bestimmen zu können. Außerdem soll die Meßsituation z.B. bei der Kontrolle von Beinlängenänderungen, wie sie etwa im Wachstum eines Patienten auftreten, oder von Lageänderungen von Gelenkspalten bei kranken Patienten reproduziert werden können, um bei jeder Messung von gleichen Voraussetzungen und Meßbedingungen ausgehen zu können.

Diese Aufgabe wird nach der Erfindung dadurch gelöst, daß am einen Ende einer Auflageplatte für das Bein ein Anschlag für den Fuß vorgesehen ist, daß zumindest an einer Längsseite der Platte eine Führung für einen Halter und eine Meßskala angeordnet sind und daß der parallel zur Meßskala verschiebbare Halter einen Meßkopf trägt.

Der Halter besteht dabei im wesentlichen aus zwei im rechten Winkel zueinander verlaufenden Schenkeln, von denen der eine vertikal stehende Schenkel am unteren Ende einen in die Führung eingreifenden Fuß aufweist, während der andere horizontale Schenkel den Meßkopf trägt, wobei der horizontale Schenkel des Halters relativ zur Auflageplatte höhenverstellbar und/oder verschwenkbar ist. Weiter ist es vorteilhaft, die Schwenkstellung des horizontalen Halterschenkels anzuzeigen, weshalb dem senkrechten Schenkel des Halters eine Winkelmeßeinrichtung zugeordnet ist.

Die Anschlagplatte für den Fuß, die der natürlichen Winkellage des Fußes relativ zum Unterschenkel anzupassen ist, ist einstellbar, wobei die Schwenkachse der Anschlagplatte die Nullage für die Meßskala an einer oder beiden Seiten der Auflageplatte bildet. Durch die Verschiebung des Halters mit dem Meßkopf in Form eines Zeigers, einer Tastnadel oder eines Sonographen kann der Meßkopf exakt z.B. über einem Gelenkspalt positioniert werden, während an der Meßskala etwa die Länge zwischen diesem Gelenkspalt und der Fußsohle abgelesen werden kann. Die Anzeige der Meßergebnisse ist sehr genau, wobei die Werte von der Längenmeßskala neben dem Fuß des Halters oder von Winkelskalen abgelesen und festgehalten werden können. Spätere Nachmessungen sind mit vorausgegangenen Meßergebnissen vergleichbar, sofern stets bei gleicher Position des Anschlags und somit reproduzierbarer Lage des Beines gemessen wird.

Die Vorrichtung kann auf eine vorhandene Liege aufgelegt werden, wodurch sie besonders einfach anwendbar ist. Strahlungschäden, wie sie bei röntgenologischen Messungen auftreten, werden vermieden, wenn mit mechanischen Meßköfpen oder Ultraschall-Meßköpfen gearbeitet wird.

Die Erfindung wird nachstehend anhand eines in der Zeichnung dargestellten Ausführungsbeispieles näher beschrieben. Es zeigt:

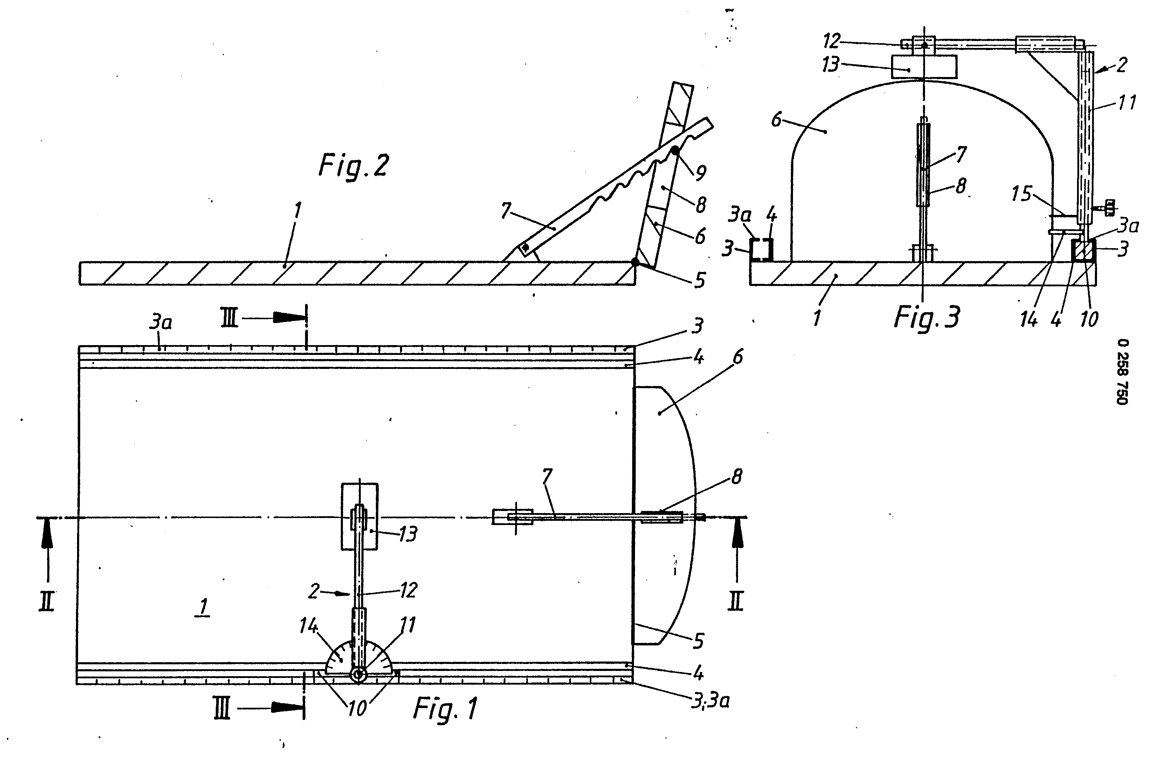

Figur 1 eine Aufsicht auf die Vorrichtung,

Figur 2 einen Längsschnitt nach Linie II-II der Figur 1 und

Figur 3 einen Querschnitt nach Linie III-III der Figur 1 in anderem Maßstab.

Die Auflageplatte 1 ist auf einer oder der Darstellung entsprechend auf beiden Längsseiten mit einer Führung für einen Halter 2 versehen. Die Führungen bestehen z.B. aus zwei U-Profilen 3 und 4 miteinander zugekehrten offenen Seiten und sind mit einer Längenmeßskala 3a kombiniert, die beispielsweise auf dem U-Profil 3 angebracht ist und deren Nullpunkt durch die Schwenkachse 5 eines Anschlages 6 für den Fuß des Patienten festgelegt ist.

In der Auflageplatte 1 ist um eine Querachse ein Hebel 7 verschwenkbar gelagert, der einen Schlitz 8 des Anschlages 6 durchgreift. Der Hebel 7 ist auf der Unterkante mit Rasten versehen, die über einen Schlitz 8 überbrückenden Zapfen 9 greifen und dadurch die einstellbare Winkellage bzw. Neigung des Anschlages 6 relativ zur Auflageplatte 1 fixieren können.

Der Halter 2 besteht aus einem Fuß 10, der in der Führung 3,4 längsbeweglich geführt ist. Mit dem Fuß 10 ist ein senkrechter, in der Länge einstellbarer Schenkel 11 verbunden. Mit dem oberen Ende des Schenkels 11 ist ein horizontaler Schenkel 12 verbunden, der einen Meßkopf 13 trägt, der beispielsweise in Form eines nach unten gerichteten Zeigers, einer Tastnadel oder eines Ultraschallwandlers ausgebildet werden kann. Wenn ein Ultraschallwandler zur Anwendung kommt, sollte dieser einen Sende-und Empfangsteil enthalten sowie in an sich bekannter Weise mit einem Monitor zusammenarbeiten, auf dem jeweils das Schnittbild des vom Schall durchstrahlten Beinbereiches bildlich dargestellt wird.

Der Halter 2 kann durch Längsverschiebung und eventuell zusätzliche Verschwenkung um die Achse des Schenkels 11 mit dem Meßkopf 13 auf den Spalt eines Fuß-, Knie-oder Hüftgelenkes ausgerichtet werden, wobei das Maß der Längsverschiebung von der Meßskala 3a beispielsweise über einen mit dem Fuß 10 verfahrbaren Zeiger und der Winkel der Verschwenkung von einem mit dem Fuß 10 starr verbundenen Winkelmeßgerät 14 und über einen mit dem Halter 2 verbundenen Zeiger 15 abgelesen werden können.

Dabei liegt das betreffende Bein des Patienten in Längs richtung auf der Auflageplatte 1 und im wesentlichen parallel zur Meßskala 3a, während die Fußsohle gegen den Anschlag 6 liegt. Es besteht natürlich auch die Möglichkeit, daß beide Beine des Patienten ausgerichtet auf der Auflageplatte 1 liegen, so daß zunächst am einen und dann am anderen Bein entsprechende Messungen durchgeführt werden können. Zu diesem Zweck kann der Halter 2 mit den an ihm befindlichen Teilen von der einen Führung 3,4 gelöst und mit der gegenüberliegenden Führung verbunden werden, falls man nicht beide Führungen mit gesonderten Haltern und Meßköpfen austatten will.

Im übrigen kann die Vorrichtung beispielsweise auf einem Tisch oder auf einer Liege abgelegt werden, so daß die Messungen an dem in einer Liegeposition befindlichen Patienten durchgeführt werden können. Andererseits ist es auch möglich, die Vorrichtung selbst als Tisch oder Liege auszubilden, wobei dann die Auflageplatte entsprechend lang ausgebildet werden muß.

### Ansprüche

1. Vorrichtung zur Bestimmung der Abmessungen eines Beines, wie beispielsweise des Abstandes zwischen Gelenkspalten und der Fußfläche, dadurch gekennzeichnet, daß an einem Ende einer Auflageplatte (1) für das Bein ein Anschlag (6) für den Fuß vorgesehen ist, daß zumindest an einer Längsseite der Auflageplatte (1) eine Führung (3,4) für einen Halter (2) und eine Meßskala (3a) angeordnet sind und daß der parallel zur Meßskala (3a) verschiebbare Halter (2) einen Meßkopf (13) trägt.

2. Vorrichtung nach Anspruch 1, dadurch gekennzeichnet, daß der Halter (2) im wesentlichen aus zwei im rechten Winkel zueinander verlaufenden Schenkeln (11,12) besteht, von denen der eine vertikal stehende Schenkel (11) am unteren Ende einen in die Führung (3,4) eingreifenden Fuß (10) aufweist, während der andere horizontale Schenkel (12) den Meßkopf (13) trägt.

3. Vorrichtung nach einem der Ansprüche 1 und 2, dadurch gekennzeichnet, daß der horizontale Schenkel (12) des Halters (2) relativ zur Auflageplatte (1) hohenverstellbar und/oder zur Achse des senkrechten Schenkels (11) verschwenkbar ist.

4. Vorrichtung nach Anspruch 3, dadurch gekennzeichnet, daß zur Anzeige der Schwenkstellung des Halters (2) eine Winkelmeßeinrichtung (14,15) vorgesehen ist.

5. Vorrichtung nach einem der Ansprüche 1 bis 4, dadurch gekennzeichnet, daß der Meßkopf (13) mindestens einen Ultraschallwandler mit einem Sende-und Empfangsteil aufweist.

6. Vorrichtung nach einem der Ansprüche 1 bis 5, dadurch gekennzeichnet, daß der Anschlag (6) über eine quer zur längsverlaufenden Meßskala (3a) liegende Achse (5) mit der Auflageplatte (1) verschwenkbar verbunden und in verschiedenen Schwenkpositionen arretierbar ist.

7. Vorrichtung nach Anspruch 6, dadurch gekennzeichnet, daß die Achse (5) des Anschlages (6) den Nullpunkt der Meßskala (3a) bestimmt.

0 258 750

Fig. 3

Fig. 2

Fig. 1